(19) Europäisches Patentamt
European Patent Office
Office européen des brevets

(11) **EP 2 581 744 A1**

(12) **EUROPEAN PATENT APPLICATION**

(43) Date of publication:
**17.04.2013 Bulletin 2013/16**

(51) Int Cl.:
***G01N 33/543*** (2006.01)    ***G01N 33/86*** (2006.01)
***G01N 33/49*** (2006.01)

(21) Application number: **11187673.6**

(22) Date of filing: **03.11.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **14.10.2011 US 547130 P**

(71) Applicant: **Koninklijke Philips Electronics N.V.
5621 BA Eindhoven (NL)**

(72) Inventor: **The designation of the inventor has not yet been filed**

(74) Representative: **Jungen, Carmen
Philips Intellectual Property &
Standards GmbH
Weißhausstraße 2
52066 Aachen (DE)**

(54) **Method for detection of coagulation activity and biomarkers**

(57) The present invention relates to a method for simultaneous detection of the coagulation activity of a blood sample and of the presence and/or amount of at least one target molecule within said blood sample, comprising the steps of:

i) introducing a blood sample into a sample container comprising magnetic particles and a sensor surface, wherein said magnetic particles are functionalized with a first binding molecule, wherein said first binding molecule is attached to said magnetic particles, wherein the first binding molecule is capable of specifically binding to the at least one target molecule within said blood sample, and wherein a second binding molecule is attached to a sensor surface at the bottom of said sample container, and wherein said second binding molecule of the sensor surface is capable of specifically binding to the at least one target molecule within said blood sample;

ii) measuring the presence and/or amount of said target molecule by detecting the number of magnetic particles bound to said sensor surface; wherein the number of bound magnetic particles is directly or inversely related to the amount of said at least one target molecule present in the sample;

iii) at the same time or at different times measuring the coagulation activity of said blood sample, wherein said magnetic particles are magnetically actuated; and wherein the loss of mobility of said magnetic particles is detected by measuring the light reflected from said immobilized magnetic particles near or at said sensor surface; and

wherein the change of detected light signal is indicative of an increase of viscosity and/or a clotting of the blood sample; and

wherein the magnetic particles in steps i) to iii) are magnetically actuated using a magnetic field generator.

FIGURE 1

EP 2 581 744 A1

## Description

### FIELD OF THE INVENTION

The present invention relates to a method for simultaneous detection of the coagulation activity of a blood sample and of the presence and/or amount of at least one target molecule within said blood sample, comprising the steps of:

> i) introducing a blood sample into a sample container comprising magnetic particles and a sensor surface, wherein said magnetic particles are functionalized with a first binding molecule, wherein said first binding molecule is attached to said magnetic particles, wherein the first binding molecule is capable of specifically binding to the at least one target molecule within said blood sample, and wherein a second binding molecule is attached to a sensor surface at the bottom of said sample container, and wherein said second binding molecule of the sensor surface is capable of specifically binding to the at least one target molecule within said blood sample;
> ii) measuring the presence and/or amount of said target molecule by detecting the number of magnetic particles bound to said sensor surface; wherein the number of bound magnetic particles is directly or inversely related to the amount of said at least one target molecule present in the sample; iii) at the same time or at different times measuring the coagulation activity of said blood sample, wherein said magnetic particles are magnetically actuated; and
> wherein the loss of mobility of said magnetic particles is detected by measuring the light reflected from said immobilized magnetic particles near or at said sensor surface; and
> wherein the change of detected light signal is indicative of an increase of viscosity and/or a clotting of the blood sample; and

wherein the magnetic particles in steps i) to iii) are magnetically actuated using a magnetic field generator.

### BACKGROUND OF THE INVENTION

The demand for universal and effective healthcare is causing the world of *in vitro* diagnostics to seek integrated random-access and point-of care solutions. The demands on such solutions are great: the tests needs to be rapid, sensitive, quantitative and accurate. Moreover the platform on which the test is performed needs to be easy to use and compact.

Thrombosis is a clot formation within a cardiac chamber or vascular lumen. Thrombosis is the principal cause of acute ischemic cardiovascular death due to occlusion of coronary or carotid arteries, also referred to arterial thromboembolism, leading to myocardial infarction and stroke, respectively. Thrombosis in the venous system is also an important cause of acute death due to pulmonary embolism. For instance, in the Netherlands annually 30,000 patients suffer a deep venous thrombosis and in 50% of these patients pulmonary embolism occurs.

Thrombosis occurs by an interaction of three elements, namely blood flow, vessel wall (damage) and blood composition. Blood coagulation is the principal mechanism that leads to the formation of a blood clot or thrombus. Since the early days of research in the field of blood coagulation, clotting tests have been established and designed to monitor clotting velocity of blood as a measure for hemostatic defects such as in haemophilia, i.e. low levels or a lack of a blood plasma clotting factor, or von Willebrand's disease, which is a defect in primary hemostasis. This type of coagulation assay comprises global clotting assays including activated partial thromboplastin time (APTT), prothrombin time (PT, translated into International Normalized Ratio, INR) and bleeding time for hemostasis.

Typically, the assays for activation products of blood coagulation which also include markers of activated platelets such as $\beta$-thromboglobulin ($\beta$-TG) have been instruments of major interest in clinical research for studying the mechanisms of thrombosis, but have been proven to be unsuitable for clinical application, mainly because these tests are rather laborious and required expensive reagents and devices. The D-dimer-test may be considered as an exception, because point-of-care (POC) tests have been developed to monitor patients suspected of venous thromboembolism. Recently, attempts have been made to stratify according to different cut-off levels. Also documentation of an increase in D-dimer level after cessation of anticoagulation may become of value in estimating the risk of recurrent venous thromboembolism

Different measuring techniques using magnetic objects for monitoring the mobility by optical means have been proposed. For example, US patent 4876069 describes a blood clotting time measuring apparatus and a method for determining and measuring the blood clotting time. The measurement of clotting time is based on a magnetic stirrer and detection by a photooptical turbidity device to monitor alterations in the movement of the stirrer, which is indicative of the mobility of the liquid. WO 99/67630 relates to methods for performing fibrinogen assays using dry chemical reagents containing Ecarin and magnetic particles. In particular, this international application describes a coagulation monitoring system using dry-chemistry reaction slides for fibrinogen detection, which optically measures the magnitude of magnetic particle oscillation.

An important drawback of these assays, however, is the fact that the means and methods known in the art are specifically tailored and limited to the measurement of a single factor of coagulation activity, namely the blood clotting time of a blood sample.

There is thus a need to design novel diagnostic strategies, which opens up possibilities for a global and integrated diagnostic evaluation. There is a strong need to provide a method to monitor the coagulation activity as a whole. Besides the clotting time, a suitable diagnostic test should also provide information on the activity of the individual factors and the history of the coagulation (e.g. D-Dimer) as a measure for coagulation activity as a whole. From the clinical viewpoint, the latter factors, also referred to as Biomarker, are also relevant for a comprehensive diagnosis.

Hence, the state of the art lacks means and methods capable of conducting an integrated evaluation combining the detection of one or more target molecules with the measurement of coagulation activity. Most favourably, such detection assays should be based on the same detection principle, and preferably may be carried out on the same device. There is thus a strong need to provide a suitable and reliable detection system capable of assaying a multitude of biochemically unrelated processes and factors for diagnosis which at the same time satisfies the requirements of an accurate and sensitive point-of-care test.

OBJECTS AND SUMMARY OF THE INVENTION

The present invention addresses these needs and provides means and methods for simultaneously measuring coagulation activity by monitoring viscosity of a blood sample within the same platform that can be also used to detect various target molecules within a blood sample.

The above objective is in particular accomplished by a method for simultaneous detection of the coagulation activity of a blood sample and of the presence and/or amount of at least one target molecule within said blood sample, comprising the steps of introducing a blood sample into a sample container comprising magnetic particles and a sensor surface, wherein said magnetic particles are functionalized with a first binding molecule, wherein said first binding molecule is attached to said magnetic particles, wherein the first binding molecule is capable of specifically binding to the at least one target molecule within said blood sample, wherein a second binding molecule is attached to a sensor surface at the bottom of said sample container, and wherein said second binding molecule of the sensor surface is capable of specifically binding to the at least one target molecule within said blood sample; measuring the presence and/or amount of said target molecule by detecting the number of beads bound to said sensor surface; wherein the number of bound beads is directly or inversely related to the amount of said at least one target molecule present in the sample; at the same time or at different times measuring the coagulation activity of said blood sample, wherein said magnetic particles are magnetically actuated; and wherein the loss of mobility of said magnetic particles is detected by measuring the light reflected from said immobilized magnetic particles near or at said sensor surface; and wherein the drop of detected light signal is indicative of an increase of viscosity and/or a clotting of the blood sample; and wherein the magnetic particles are magnetically actuated using a magnetic field generator.

One major advantage is that the different measurements can now be carried out on the same detection platform, although their underlying biochemistry and physics are very different. It will be appreciated that such a combinatorial measurement offers several advantages for clinical practice. For instance, only a small blood sample from an individual or patient is sufficient to accurately carry out a complete test within only a short period of time. The combinatorial detection method according to the present invention is thus most convenient, mainly because a single sample from an individual or a patient is required, which may serve for a multitude of assaying steps. Hence, it is no longer required that several samples have to be subjected to different diagnostic tests, different test devices or even to different laboratories in order to obtain a global picture for diagnosis.

Differential processing of a sample is also not required with enormous saving of costs and time. The method according to the invention is thus capable of immediately providing sufficient and accurate results, thus facilitating correct conclusions regarding suitable medication. The method as described herein thus meets the high demands of modem point-of-care solutions.

In particular, the examples of the present application demonstrate that coagulation activity of a given sample can be accurately measured using such an integrated assay. It could be shown that the loss of mobility of magnetic particles functionalized with a binding molecule suited for the detection of one or more target molecules within the sample to be tested can be advantageously used for such an assay. Moreover, the application demonstrates that that the detection of clotting time can be conducted on the same optical detection system.

The Examples of the present application also show that a change in light signal derived from immobilized magnetic particles near or at the bottom surface of the sample container may serve as an accurate measure for clotting time of a blood sample.

Without wishing to be bound to a theory one explanation for the observed feasibility of the method according to the present invention is that only a small evanescent area near or at the bottom surface of the sample container is used for the detection of the loss of mobility of the magnetic particles. Contrary to a measurement of the whole sample volume as described in the art, one further advantage is thus that such a restriction to an evanescent detection field minimizes the effects derived from the bulk content, which could otherwise negatively influence detection accuracy. For instance, unspecific binding or clustering of the beads is thereby minimized.

Finally, one major advantage over the art can be seen in the fact that at the same or at different times, that means shortly before or after the detection of coagulation activity, one or more optomagnetic detection assays can be additionally applied, which can measure the presence and/or amount of at least one or more target molecules within the blood sample to be tested.

In a preferred embodiment of the present invention the detection of the coagulation activity of a blood sample and of the presence and/or amount of at least one target molecule within said blood sample is carried out using frustrated total internal reflection (FTIR).

In a further preferred embodiment of the present invention all steps are carried out in the same sample container.

In a further preferred embodiment of the present invention all steps are carried using the same magnetic particles, the magnetic particles preferably being superparamagnetic beads.

In another preferred embodiment of the present invention the first binding molecule is an antibody or a fragment thereof.

In a further preferred embodiment of the present invention the blood sample is taken from an individual, wherein the blood sample is an untreated whole blood sample or a blood-derived sample.

In another preferred embodiment of the present invention the magnetic particles are actuated in such a way that the beads are pulled towards and away from the surface in a pulse like fashion.

In further preferred embodiment of the present invention the light reflected from the magnetic particles is measured over time, wherein introduction of the blood sample is designated as the starting point and said drop of detected light signal is designated as the endpoint, and wherein the period between said starting point and end point correlates to the clotting time.

In yet another preferred embodiment the coagulation activity is induced by the addition of a further substance into the sample container.

In another preferred embodiment of the present invention the at least one target molecule is a biomarker for diagnosis and/or course of diseases.

In another preferred embodiment of the present invention the biomarker is selected from the group selected from nucleic acids, proteins and metabolites.

In another preferred embodiment of the present invention the biomarker molecule is a coagulation factor.

In a further preferred embodiment of the present invention the second binding molecule is an antibody or a fragment thereof.

In yet a further preferred embodiment of the present invention the presence and/or amount of the at least one target molecule is detected using a sandwich immunoassay.

BRIEF DESCRIPTION OF THE DRAWINGS

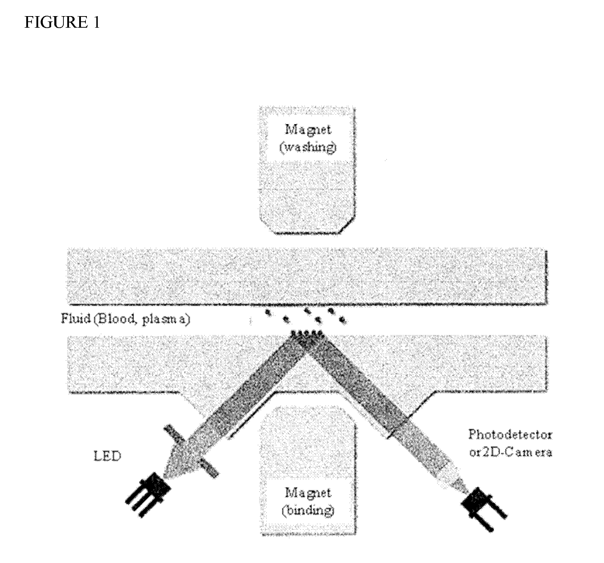

Fig. 1 shows as schematic representation (side view) of an exemplary optomagnetic apparatus suitable for simultaneous measurement of coagulation activity of a given sample and presence and/or amount of a target molecule within a blood sample. Typically, the coagulation activity is measured in a sensor device, wherein magnetic particles within a cartridge or sample container are detected by frustrated total internal reflection (FTIR). Such an apparatus typically comprises a light source (LED) emitting an input light beam, a light detector (photodetector) for detecting and measuring an output light beam, and connected thereto an evaluation unit. The input light beam is emitted into a cartridge or sample container made from glass or a transparent plastic. The cartridge or container comprises the fluid sample to be tested. The sample further comprises magnetic particles, for instance, superparamagnetic beads, which can be functionalized with a binding molecule for detection of a target molecule and which can be magnetically actuated by an upper (washing magnet) and a lower magnet (binding magnet). The presence of magnetic beads at a sensor surface in the sample container can be detected using such an optomagnetic sensor system.

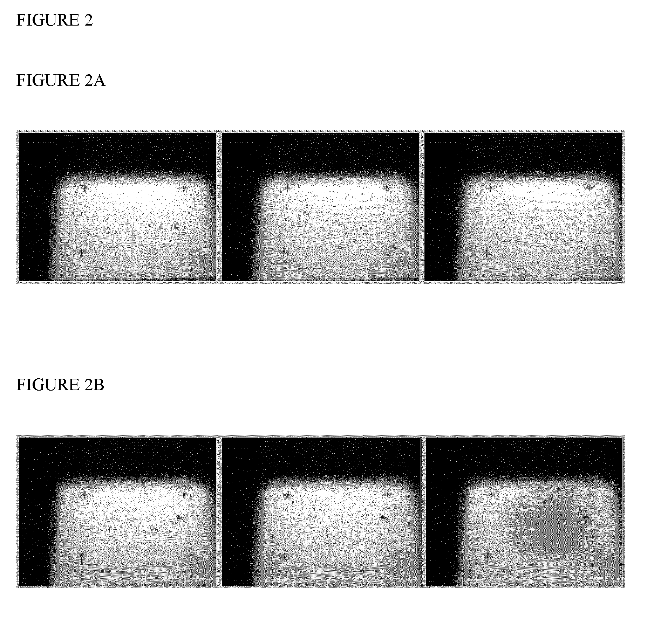

Fig. 2 shows the FTIR images resulting from the experiment as described in Example 1 using the experimental setup as shown in Fig. 1. Fig. 2a (upper panel) shows FTIR images of a selected evanescent field at the bottom surface of the sample chamber without the addition of calcium at time points 0 (left panel), 3 (middle panel) and 7 minutes (right panel). The magnetic beads can move freely towards and away from the depicted surface due to magnetic actuation when no calcium is added, i.e. formation of fibrin is not critical for the movement of the beads. Fig. 2b shows the respective FTIR images, however, with the addition of 0.125 M $CaCl_2$ to the citrate plasma at time points 0 (left panel), 3 (middle panel) and 7 minutes (right panel). The right panel shows enhanced gray signal deriving from magnetic beads that are compromised in their mobility at or close to the surface leading to a contrast enhancement in the FTIR image.

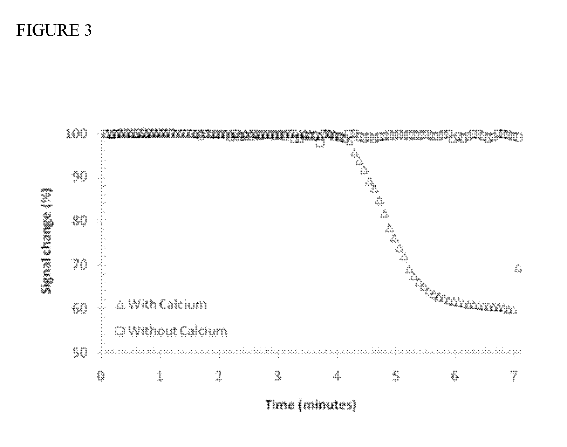

Fig. 3 is a diagram showing the measurement of signal change (%) over time obtained in the experiment as described in Example 1. Squares correspond to data points from the measurement without the addition of calcium (control) whereas triangles correspond to data points from the experiment with calcium addition. While in the measurement without the addition of calcium the light signal remains unchanged over time, the curve corresponding to the addition of calcium shows a strong decrease of light signal at about 4 minutes. Within a minute the change in light signal decreases to a value about 60%.

DETAILED DESCRIPTION OF THE EMBODIMENTS

The present invention relates to a novel method for simultaneous detection of the coagulation activity of a blood sample and of the presence and/or amount of at least one target molecule within a given blood sample.

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of $\pm 20$ %, preferably $\pm 15$ %, more preferably $\pm 10$ %, and even more preferably $\pm 5$ %.

It is to be understood that the term "comprising" is not limiting. For the purposes of the present invention the term "consisting of" is considered to be a preferred embodiment of the term "comprising of". If hereinafter a group is defined to comprise at least a certain number of embodiments, this is meant to also encompass a group which preferably consists of these embodiments only.

Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In case the terms "first", "second", "third" or "(a)", "(b)", "(c)", "(d)" "i", "ii" etc. relate to steps of a method or use or assay there is no time or time interval coherence between the steps, i.e. the steps may be carried out simultaneously or there may be time intervals of seconds, minutes, hours, days, weeks, months or even years between such steps, unless otherwise indicated in the application as set forth herein above or below.

It is to be understood that this invention is not limited to the particular methodology, protocols, reagents etc. described herein, as these may vary. It is also to be understood that the terminology used herein is for the purpose of describing particular embodiments only, and is not intended to limit the scope of the present invention that will be limited only by the appended claims. Unless defined otherwise, all technical and scientific terms used herein have the same meanings as commonly understood by one of ordinary skill in the art.

As has been set out above, the present invention concerns a method for simultaneous detection of the coagulation activity of a blood sample and of the presence and/or amount of at least one target molecule within said blood sample, comprising the steps of:

i) introducing a blood sample into a sample container comprising magnetic particles and a sensor surface, wherein said magnetic particles are functionalized with a first binding molecule, wherein said first binding molecule is attached to said magnetic particles, wherein the first binding molecule is capable of specifically binding to the at least one target molecule within said blood sample, and wherein a second binding molecule is attached to a sensor surface at the bottom of said sample container, and wherein said second binding molecule of the sensor surface are capable of binding said at least one target molecule;

ii) measuring the presence and/or amount of said target molecule by detecting the number of beads bound to said sensor surface; wherein the number of bound beads is directly or inversely related to the amount of said at least one target molecule present in the sample;

iii) at the same time or at different times measuring the coagulation activity of said blood sample, wherein said magnetic particles are magnetically actuated; and

wherein the loss of mobility of said magnetic particles is detected by measuring the light reflected from said immobilized magnetic particles near or at said sensor surface; and

wherein the drop of detected light signal is indicative of an increase of viscosity and/or a clotting of the blood sample; and

wherein the magnetic particles in steps i) to iii) are magnetically actuated using a magnetic field generator.

The term "simultaneous detection as used herein means that both measurements, i.e. the detection of the coagulation activity of a blood sample and detection of the presence and/or amount of at least one target molecule within a blood sample can be carried out at the same or at different times.

The term "at different times" as used herein means that one of the detection methods is carried out before or after the second detection method. For instance, in an illustrative example and particularly preferred embodiment of the present invention the detection of the presence and/or amount of a biomarker is carried out before detection of coagulation activity via determination of clotting time. It is also conceivable that the chronological order of detection may be reversed, i.e. the detection of the presence and/or amount of a biomarker is carried out shortly after measurement of coagulation activity.

Within the meaning of the present invention "simultaneous detection" also implies that the both detection assays are carried using the same optomagnetic device. For instance, it is envisaged by the present application that such a simultaneous detection can be carried out by using various cartridges or sample containers, each comprising a different set of magnetic particles for the performance on the same biosensor device. Alternatively, the blood sample to be tested may be comprised within in the same sample container or cartridge so that a single sample is subjected to multiple detection steps or assays. In such a case, the one sample container may contain one or more different magnetic particles for detection of one or more target molecules within the blood sample. The simultaneous detection as described in the present application thus offers a convenient assaying method for measuring a plurality of diagnostic factors in a single sample within a short period of time based on the same biosensor platform.

The term "coagulation activity" as used herein refers to the activity related to the clotting function of blood or blood-derived samples. In principle, the formation of a blood clot or thrombus occurs when the proteins of the coagulation cascade are activated either by contact with damaged blood vessel wall (also referred to as intrinsic pathway) or by activation of factor VII by tissue activating factors (extrinsic pathway). The intrinsic and extrinsic pathway lead to formation of the enzyme thrombin, which is capable of converting the soluble blood protein fibrinogen into fibrin which further aggregates into proteofibrils.

Another important enzyme, namely factor XIII, then functions to crosslink the fibrin proteofibrils at the D-fragment site resulting in the establishment of an insoluble gel which serves as a scaffold for blood clot formation.

In clinical practice, coagulation factor levels are typically used to determine coagulation activity, i.e. whether the level of the factor is associated with reduced clot formation and bleeding, or with increased clot formation such as thrombosis. Normal coagulation factor activity usually implies that the clotting function is normal. Low activity of one or more coagulation factors usually means impaired clotting ability. Deficiencies in coagulation factors may be acquired or inherited, e.g. Hemophilia A and B. Elevated levels of several factor are often observed in states of acute illness, stress or inflammation. For instance, the persistent high levels of factor VIII may be associated with an increased risk of venous thrombosis.

In addition, also the clotting time can be advantageously used as a measure for coagulation activity. The term "clotting time" as used in the context of the present invention can be generally defined as the time required for the formation of a fibrin clot. The coagulation assays described in the art including prothrombin time (PT), partial thromboplastin time (PTT), activated partial thomboplastin time (APTT), fibrinogen assay, thrombin clotting time (TCT), activated clotting time (ACT) are directly or indirectly based on clotting time measurements.

In the method according to the present invention, the mobility of the magnetic particles used in the assay for coagulation activity is detected and monitored over time. In particular, the loss of mobility of the magnetic particles can be detected by constantly measuring the light reflected from an evanescent area of the sensor surface. The clotting time assay as described herein thus exploits the fact that loss of mobility of the magnetic particles leads to a change in detected light, which could be shown to inversely correlate to an increase in the viscosity of a blood sample, especially to an increase in the density of fibrin network. The detection of immobilized magnetic particles at a sensor surface can thus advantageously be used as direct measure for clot formation.

Typically, the end point of clot formation is reached when fibrinogen is maximally polymerized to the fibrin coagulum. The resulting high viscosity and/or the increased density of the fibrin network leads to the entrapment of the magnetic particles within the sample, i.e. a complete loss of mobility of the magnetic particles.

Using the method according to the invention the loss of mobility of the magnetic particles can be made directly visible by monitoring the light reflected from the particles. A further advantage of the coagulation activity assaying step according to the invention is that only immobilized magnetic particles near or at the sensor surface exhibit a change in light signal.

In particular preferred embodiments of the present invention the coagulation activity of a blood sample is detected based on frustrated total internal reflection (FTIR).

A "target molecule" as used herein may be any molecule bound by a binding molecule and may for example be a biological substance such as a biomolecule, complexes, cell fractions or cells. Preferably, a target molecule within the context of the present invention is a nucleic acid, e.g. DNA, or RNA molecule or an oligonucleotide such as a DNA or RNA oligonucleotide. Even more preferred are target molecules such as a peptide, a protein, a drug molecule, a small molecule, or a vitamin. In particular preferred embodiments of the present a "target molecule" may be a biomarker molecule.

A "magnetic particle" as used herein means a small, localized object to which can be ascribed a physical property such as volume or mass. The term "magnetic particles" shall comprise both permanently magnetic particles as well as magnetisable particles, for example, superparamagnetic particles. Furthermore, a particle essentially behaves as a whole unit in terms of its transport and properties. Magnetic particles may accordingly be of a symmetrical, globular, essentially globular or spherical shape, or be of an irregular, asymmetric shape or form.

The size of a magnetic particle envisaged by the present invention typically ranges between 3 nm and 50 $\mu$m. Preferred are magnetic particles in the nanometer and micrometer range up to several micrometers. Particularly preferred are magnetic nanoparticles, e.g. particles with a diameter of about 1 to 700 nanometers. Particularly preferred are nanoparticles which may have a diameter of about 10 to 600 nm, e.g. 10 nm, 15 nm, 20 nm, 25 nm, 30 nm, 35 nm, 40 nm, 45 nm, 50 nm, 60 nm, 70 nm, 80 nm, 90 nm, 100 nm, 120 nm, 150 nm, 170 nm, 200 nm, 220 nm, 250 nm, 270 nm, 300 nm, 320 nm, 350 nm, 370 nm, 400 nm, 420 nm, 450 nm, 470 nm, 500 nm, 520 nm, 550 nm, 570 nm, 600 nm, 620 nm or any value in between. Even more preferred are nanoparticles having a diameter of about 500 nm.

Typically, the magnetic particles according to the present invention are functionalized with a first binding protein. The term "first binding molecule" as used herein refers to any molecule having a high binding affinity for a second molecule, i.e. an interaction partner. A binding molecule in the sense of the present invention typically comprises binding or capture moieties capable of binding a specific target molecule, preferably a biomarker, or capable of binding a molecule-containing target entity, such as for example a virus, or a cell or a cell fragment, or material derived from tissue.

In a particularly preferred embodiment, the binding molecule is selected from the group consisting of aptamers, peptides, proteins, oligonucleotides, and molecular imprinted polymers, wherein said affinity molecule preferably is an antibody or a fragment thereof.

An "aptamer" as used within the context of an affinity molecule may be a short nucleic acid molecule, e.g. an RNA, DNA, PNA, CNA, HNA, LNA or ANA molecule or any other suitable nucleic acid format known to the person skilled in the art, being capable of binding to a target molecule as defined herein, preferably to a nucleic acid target molecule as defined herein. Furthermore, the present invention envisages peptide aptamers, i.e. aptamers which are able to specifically bind to (a) protein(s), polypeptide(s) or peptide(s) comprising a specific amino acid sequence(s). Typically, (a) peptide aptamer(s) is/are a variable peptide loop(s), comprising for example 10 to 20 amino acids. In the context of the present invention the peptide aptamer(s) may in specific embodiments be attached at one or both ends to a scaffold structure. The scaffold structure may be any molecule, preferably a protein, e.g. a protein, which has good solubility properties. Suitable scaffold molecules would be known to the person skilled in the art. An example of suitable scaffold molecule to be used in the context of the present invention is the bacterial protein thioredoxin-A. The aptamer peptide loop may preferably be inserted within a reducing active site of the scaffold molecule. Alternatively, staphylococcal protein A and domains thereof and derivatives of these domains, such as protein Z or lipocalins may be used as scaffold structures in the context of the present invention. Nucleic acid or peptide aptamers may be generated according to any suitable method known to the person skilled in the art, e.g. via PCR or molecular synthesis approaches or yeast two-hybrid approaches.

A "peptide" as used within the context of an affinity molecule may comprise or alternatively consist of a stretch of 2 to 35 amino acids, amino acid derivatives or a mixture thereof. The peptide may be linear, branched, circular or mixture thereof. A peptide affinity molecule may also be attached to a scaffold structure as defined herein above.

A "protein" as used within the context of an affinity molecule may comprise or alternatively consist of a stretch more than about 35 amino acids, amino acid derivatives or a mixture thereof. The protein may have a linear, branched, circular form or be comprised of a mixture of these forms. A protein affinity molecule may also be attached to a scaffold structure as defined herein above.

An "oligonucleotide" as used within the context of an affinity molecule may comprise or alternatively consist of a stretch of about 5 to 120 nucleotides, e.g. a stretch of 10, 20, 30, 40, 50, 60, 70, 80, 90 or 100 nucleotides, preferably of about 15 to 60 nucleotides. An oligonucleotide affinity molecule may preferably be an RNA molecule or a DNA molecule, or a mixture of both.

The term "molecular imprinted polymer" as used herein refers to a polymer which was formed in the presence of a molecule that is extracted afterwards, leaving complementary cavities behind. Typically, a molecular imprinted polymer shows a certain chemical affinity for the original molecule. A molecular imprinted polymer may be composed of any suitable polymeric unit known to the person skilled in the art. Techniques for their production include polymerization techniques such as bulk, precipitation, emulsion, suspension, dispersion, gelation, and multi-step swelling polymerization. Particularly preferred are hierarchical imprinting methods.

An "antibody" as used within the context of a binding molecule refers to an immunoglobulin molecules and immunologically active portions of immunoglobulin molecules, i.e. molecules that contain an antigen binding site that immunospecifically binds an antigen. The immunoglobulin molecules of the invention can be of any type (e. g., IgG, IgE, IgM, IgD, IgA and IgY), class (e. g., IgG1, IgG2, IgG3, 1gG4, 1gA1 and IgA2) or subclass of immunoglobulin molecules. Antibodies of the present invention may be described or specified in terms of the epitope(s) or portion(s) of a polypeptide of the present invention which they recognize or specifically bind. Specific epitopes and their interaction with antibodies would be known to the person skilled in the art. The term "specifically binding" as used herein refers to the immunospecific detection and binding of an antibody to an antigenic epitope. The term "specifically binding" excludes non-specific binding but does not necessarily exclude cross-reactivity with other antigens, in particular with antigens comprising the same antigenic epitope detected by the present antibody.

The antibody may be a polyclonal, monoclonal, multispecific, human, humanized or chimeric antibody, single chain antibody, or constitute a Fab fragment, Fab' fragment, a fragment produced by a Fab expression library, F(ab')2, Fv, disulfide linked Fv, minibody, diabody, scFv, sc(Fv)2, whole immunoglobulin molecule, small modular immunopharmaceutical (SMIP), binding-domain immunoglobulin fusion protein, camelized antibody, $V_{HH}$ containing antibody, an anti-idiotypic (anti-Id) antibody an any epitope-binding fragment(s) of any of the above. Most preferably, the antibodies are human antigen-binding antibody fragments of the present invention and include Fab, Fab' and F (ab')2, Fv, single-chain Fvs (scFv), sc(Fv)2, single-chain antibodies, disulfide-linked Fvs (sdFv) and fragments comprising either a VL or VH domain.

The antibodies according to the invention may be of any animal origin including birds and mammals. Preferably, the antibodies are human, murine (e. g., mouse and rat), donkey, monkey, rabbit, goat, guinea pig, camel, horse, or chicken antibodies.

The antibodies according to the present invention may be monospecific, bispecific, trispecific or of greater multispecificity. Multispecific antibodies may be specific for different epitopes of a polypeptide of the present invention or may be specific for both a polypeptide of the present invention as well as for a heterologous epitope, such as a heterologous polypeptide or solid support material. Preferred are monospecific antibodies.

As used herein, the term "change in light signal" means a difference in light reflected from the magnetic particles which is detected by optical means. For instance, such methods may include methods such as the detection of scattered light or detection based on total internal reflection (TIR) or frustrated total internal reflection (FTIR). Preferably, the change in light signal refers to only those magnetic particles being close or at the sensor surface. Such a measurement typically requires that the magnetic particles within the area of detection are hampered in their movement, i.e. have become substantially immobilized or have entirely lost mobility. Typically, the inventive method for clotting time measurement is based on a technique sensitive to the presence of particles at the surface, preferably frustrated total internal reflection (FTIR). A change in light signal is preferably measured as a decrease in light signal and calculated as signal change (%). The signal change may have a value of less than 90%, preferably less than 85, 84, 83, 82, 81, and 80%, more preferably less than 79, 78, 77, 76, 75, 74, 73, 72, 71, and 70 %, even more preferably less than 69, 68, 67, and 66%, and most preferably less than 65, 64, 63 62, 61, and 60% to be indicative of the loss of movement of the magnetic particles.

As used herein the term "total internal reflection" describes a condition present in certain materials when light enters one material from another material with a higher refractive index at an angle of incidence greater than a specific angle. The specific angle at which this occurs depends on the refractive indices of both material, also referred to as critical angle and can be calculated mathematically (Snell's law, law of refraction). In absence of magnetic particles no refraction occurs and the light beam from the light source is totally reflected. If a magnetic particle is close to the surface or is in contact with the surface the light rays are said to be frustrated by the particle and reflection at that point is no longer total.

The signal, which may be defined as the decrease of the totally internal reflected signal, can be calculated. The signal is more or less linearly dependent on the concentration of beads on the surface (surface density n̄). The signal can be expressed as:

$$S = \beta \, \tilde{n},$$

wherein S is the measured signal change in % and $\beta$ i a conversion factor from surface density to signal change.

A "sample" as used herein refers to any sample, preferably a blood sample which includes a target molecules as defined herein. Such samples may, for example, include samples taken from an individual or a patient. The blood sample may be whole blood, the whole blood sample can be untreated or treated, or can be a blood-derived samples such as serum, plasma, citrated plasma or any blood plasma, which has been treated with an anticoagulant. It is also envisaged by the present invention to use a diluted blood plasma with a buffer where appropriate for the assays according to the invention. Suitable buffers for the dilution of blood are known to the skilled person and include, for instance, Owrens buffer, HEPES, TRIS or PBS.

The term "blood plasma" as used herein is defined as the yellow liquid component of blood in which the blood cells in whole blood are normally suspended, whereas serum normally refers to is blood plasma without fibrinogen or other clotting factors. Furthermore, antibiotics or bactericides may be added to samples to prevent further growth of any organisms present. Whole cells may also be removed or may be lysed to release their contents.

The term "citrated plasma" as used herein refers to blood which is treated with sodium citrate to prevent blood clotting. Citrate thus exhibits anticoagulant activity in blood plasma which can be reversed by increase of calcium concentration.

An "anticoagulant" used herein refers to a substance capable of preventing coagulation, i.e. blood clotting. Anticoagulants include but are not limited thereto, for instance, EDTA, citrate, oxalate, aspirin, clopidogrel, dipyridamole and ticlopidine, parenteral glycoprotein IIb/IIIb inhibitors, Warfarin and related coumarins such as Acenoumarol, phenprocoumon, Brodifacoum, and Phenindione, Heparin and derivatives thereof, synthetic pentasaccharide inhibitors of Xa such as Fondaparinux and Idraparinux, direct thrombin inihibitors such as argatroban, lepirudin, bivalirudin and dabigatran, direct Xa inhibitors such as rivaroxaban and apixaban and novel anticoagulant drugs such as Batroxobin and Hementin. Anticoagulants can be also used as medication for thrombotic disorders. It is common in clinical practice to use anticoagulants in medical equipment such as test tubes, blood transfusion bags and dialysis. It is appreciated that were appropriate anticoagulants may thus be present in a blood sample in order prevent immediate clotting or for transport or storage purposes.

In some embodiments, target molecules such as biomarker are directly obtained from samples. In other situations samples may be first subjected to sample preparation techniques, e.g. based on standard protocols, including, for example, partial purification, which renders the target molecules more accessible to binding partners, i.e. a first and second binding molecule as defined herein. For example, blood samples may be centrifuged to separate fractions including whole cells or membranes from plasma or serum.

A "sample container" or "sample chamber" as used herein refer to a container made from glass or any transparent plastic in which the blood sample is measured. The magnetic particles as described herein may be already present in the sample container, introduced together with the blood sample, or introduced after the blood sample has been injected into the sample container. The sample container further comprises a contact or sensor surface. Typically, the sensor surface is at the bottom of the sample container. The sample container may further be located in an exchangeable cartridge, i.e. in a standalone component separate from the sensor device. Due to possible contamination with a sample, such a cartridge will usually be a disposable item, made for instance from plastics by injection moulding.

A "sensor surface" as used herein, defines an area for detection and may be typically located the bottom of the sample container. The sensor surface may serve for the detection of the presence and/or amount of a target molecule within a blood sample. As such, the sensor surface typically comprises one or more sub-regions. For instance, one sub-region may be adapted to detect the presence of magnetic particles at the surface, also referred to as "target region". The sensor surface may further comprise one or more sub-regions called "reference region". For such a detection of at least one target molecule, the sensor surface may comprise a second binding molecule, which can be directly or indirectly, i.e. via a spacer or linker, attached to the one or more sub-regions of the sensor surface. Typically, the sensor surface may generate an optical image comprising both the target region and the reference region(s), wherein separate parts of this image are individually processed.

The sensor surface may be also used for the measurement of coagulation activity via clotting time detection. In this case, the sensor surface or any defined sub-region of the sensor surface may be used to measure the mobility of magnetic particles in a blood sample to be tested. In the clotting time assay as described herein, the sensor surface may generate an optical image, wherein the loss of mobility of magnetic particle is visible in the image as a change in light signal. Preferably, the change in light signal is caused by immobilized or trapped magnetic particle present near or at the sensor surface. For such a measurement, the binding functions of the first binding molecule present on the magnetic particle as well as the second binding molecules attached to the surface of the sensor surface are irrelevant. Preferably, the sub-region defined for the measurement of coagulation activity is selected such that this sub-region does not overlap with the one or more sub-regions for detection of the presence/or amount of at least one target molecules.

Typically, the simultaneous detection as described herein may be carried out in a optomagnetic biosensor device. Suitable devices for the detection method according to the present invention have been previously disclosed, for instance, in WO 2008/155716.

In particular preferred embodiments of the present invention, the Magnotech® biosensor system is used. It will be however appreciated that the detection principle underlying the present invention is not restricted to the Magnotech® system. In principle, also any other optomagnetic detection system may be used which satisfies the minimal criteria as depicted in Figure 1. Typically, a suitable optomagnetic system may comprise a light source (LED) emitting an input light beam, a light detector (photodetector) for detecting and measuring an output light beam, and connected thereto an evaluation unit. The device further comprises a cartridge or sample container for introducing and measuring a blood sample. The sample container may further comprise magnetic particles which can be functionalized with a binding molecule such as an antibody for detection of one or more target molecules within the blood sample. Typically, the optomagnetic device comprises at least two magnets for magnetic actuation of the beads. In an exemplary arrangement, the biosensor device may comprise a magnetic field generator as described herein, preferably comprising an upper (washing magnet) and a lower magnet (binding magnet). The presence of magnetic beads at a sensor surface in the sample container can be detected using such an optomagnetic sensor system.

The measurement of "presence and/or amount of at least one target molecules" using such a biosensor device has been described in previous applications, for instance, in EP 1801594 A and WO 2007/010469 A1. Typically, such biosensor assays can be used for rapid, sensitive and easy-to-use molecular diagnostics, especially to detect biological targets. Such an assay uses magnetic forces acting on magnetic particles functionalized with a first binding molecule capable of recognizing and binding the biological target in the sample.

In a first phase of such an assay, the magnetic particles coated with the first binding molecule move through the sample solution for effective target molecule capture. Subsequently, actuating magnets are engaged to move and transport the magnetic particles with high speed to the sensor surface for binding. Typically, attached to the sensor surface are second binding molecules also capable of binding the biological target. Thereafter, a sequence of finely tuned magnetic pulses is applied to facilitate optimal binding and mixing of the magnetic particles that have captured the target molecules. After the particles have bound to the sensor surface, free and non-specifically bound particles are rapidly removed by applying a magnetic field oriented away from the detection surface, herein also referred to as washing magnet. The presence and/or amount of the magnetic particles bound at the sensor surface can be detected optically, wherein the number of bound magnetic particles is directly or inversely related to the amount of target molecules present in the sample, preferably the optical detection is based on frustrated total internal reflection (FTIR). Preferred detection assays envisaged by the present invention are antibody-based immunoassays such as competitive and non-competitive immunoassays.

The term "competitive immunoassay" refers to immunoassays, wherein the antigen (target molecule) in the sample competes with a labeled antigen to bind with antibodies. The amount of labeled antigen bound to the antibody site is then measured. In this method, the response will be inversely related to the concentration of antibody in the sample. This means that the greater a response, the less antigen in the sample was available to compete with the labeled antigen.

The term "non-competitive immunoassay" also referred to as the "sandwich immunoassay" refers to immunoassays, wherein the antigen in the sample to be tested is bound to a first antibody or binding molecule and further detected by a second antibody or binding molecule. The amount of bound second antibody is then measured. Using the detection principle as described herein, the amount of magnetic particles bound to the second antibody or binding molecule is a suitable measure for determining the amount of antigen or target molecules. In contrast to the competitive method, the results of the non-competitive method will be directly proportional to the concentration of the antigen.

A "magnetic field generator" as used herein typically comprises one or more magnets within the optomagnetic device as described herein for generating a magnetic field with the sample chamber., wherein said magnetic field shall guide magnetic particles in their movement towards the sensor surface. The magnetic field will typically have a non-zero gradient that allows to exert magnetic forces on magnetic (dipole) particles. At the same time the magnetic field generator may also be used to create a frequent and oscillatory movement of the beads. Loss of mobility of the so magnetically actuated particles may be detected at the sensor surface and may be advantageously be used as an indirect measure for coagulation activity.

In a preferred embodiment of the present invention the detection of the coagulation activity of a blood sample and of the presence and/or amount of at least one target molecule within said blood sample is carried out using frustrated total internal reflection (FTIR).

In a further preferred embodiment of the present invention all steps are carried out in the same sample container. It is envisaged by the present invention that a single sample be subjected to different simultaneous or timely separated detection assays. For this purpose, the sample container may comprise magnetic particles which are uniformly functionalized, i.e. all magnetic particles are coated with the same first binding molecule for capturing the target. Alternatively, also the sample container may comprise at least two, at least three or several differentially functionalized beads. It is thus conceivable that one sample cartridge can be optimized to conduct a combinatorial measurement of blood clotting time with several detection several biological targets. Accordingly, the sensor surface has to be adapted to carry out such a multiple detection platform.

In a further preferred embodiment of the present invention all steps are carried using the same magnetic particles, the magnetic particles preferably be superparamagnetic beads. The term "superparamagnetic" as used herein describes a form of magnetism which appears in small ferromagnetic or ferromagnetic nanoparticles. It is known in the art that in sufficiently small nanoparticles, magnetization can randomly flip direction under the influence of temperature. The time between two flips is referred to as the Néel relaxation time. In the absence of an external magnetic field, when the time used to measure the magnetization of the nanoparticles is much longer than the Néel relaxation time, the magnetization appears to be in average zero, i.e. in the paramagnetic state. In such a state an external magnetic field is able to magnetize the nanoparticles similarly to a paramagnet. However, the magnetic susceptibility is much larger than those of paramagnets.

In another preferred embodiment of the present invention the first binding molecule is an antibody or a fragment thereof as defined herein above.

In a further preferred embodiment of the present invention the blood sample is taken from an individual, wherein the blood sample is an untreated whole blood sample or a blood-derived sample.

In another preferred embodiment of the present invention the magnetic particles are actuated in such a way that the magnetic particles are pulled towards and away from the surface in a pulse like fashion. As described herein above, for instance in Figure 1, the magnetic actuation may be carried out using a lower magnet, also referred to as binding magnet, and a upper magnet or washing magnet. While in the assay for detection of the presence and/or amount of target molecules the alternate action of these two magnets results in fine tuned pulses for the binding and washing steps, the same magnetic actuation may be advantageously applied for detection of clotting time. In some preferred embodiments the magnetic could have different strengths. For example, it is envisaged by the present invention that the magnetic particles are preferably attracted to the sensor surface. It could thus be advantageous to provide a magnetic actuation, wherein the force from the binding magnet is much greater than the washing magnet. The resulting movement of the magnetic beads can be described as a frequent and oscillatory "up and down" movement toward and away from the sensor surface.

In further preferred embodiment of the present invention the light reflected from the magnetic particles is measured over time, wherein introduction of the blood sample is designated as the starting point and the decrease of detected light signal is designated as the end point, and wherein the period between said starting point and end point correlates to the clotting time. The blood clotting process leads to formation of a fibrin coagulum. During polymerization of fibrinogen a more and more dense fibrin network is formed resulting in an increased viscosity of the blood sample. In the sense of the present invention, the end point is reached when viscosity of the blood sample and/or density of the fibrin network becomes critical for the movement of the magnetic particles, i.e. the particles are trapped or immobilized by the fibrin meshwork thus formed. Hence, the detected loss of mobility of the magnetic particles may thus be indicative of the endpoint of clotting time.

In the illustrative Examples of the present application, the loss of mobility of the magnetic particles used in this optomagnetic assay is detected using the Magnotech®-biosensor platform. Due to the creation of an evanescence field by FTIR, the magnetic beads only become visible when trapped or immobilized close to or at the sensor surface, thereby creating a high contrast in the FTIR image. In contrast, magnetic beads which are not compromised in their movement can move freely and frequently due to the magnetic actuation and do thus not result in a signal change in the FTIR image. As can be seem from Figure 2A the beads move freely through the citrate plasma in absence of calcium indicating that the formation of fibrin at each of the time points 0,3 and 7 minutes (after injection) is not critical for movement of the beads, whereas in Figure 2B the formation of a coagulum leads to restriction of this movement. The immobilized or trapped magnetic beads due to their higher contrast become visible in form of gray spot in the FTIR image.

In yet another preferred embodiment the coagulation activity is induced by the addition of a further substance into the sample container. It is also envisaged by the present invention that the addition of a substance may reverse the anticoagulant effect. In principle, any substance capable of reversing the effect of an anticoagulant can be advantageously used for activation of coagulation of blood plasma where clotting is prevented by the presence of an anticoagulant as described herein above. For instance, in a particularly preferred examples of the present invention, calcium may be added to citrated blood plasma in order to activate blood coagulation. Preferably, such a substance is added prior or at the starting point of the measurement. It is also conceivable that the non-addition may be advantageously used in a control, which is representative for a state where coagulation does not occur.

In another preferred embodiment of the present invention the at least one target molecule is a biomarker for diagnosis and/or course of diseases. The term "biomarker" as used herein can be defined as a characteristic that is objectively measured and evaluated as an indicator of normal biological processes, pathogenic processes or pharmacologic responds to a therapeutic intervention. The biomarker molecules envisaged by the present invention can be any biomarker known in the art such as disease-related biomarker or drug-related biomarker. Disease-related biomarkers provide an indication of the probable effect of treatment on patient (risk indicator or predictive biomarkers), if a disease already exists (diagnostic biomarker), or how such a disease may develop in an individual case regardless of the type of treatment (prognostic biomarker). Predictive biomarkers generally helps to assess the most likely response to a particular treatment, whereas prognostic markers are used to monitor the progression of a disease with or without treatment. In contrast, drug-related biomarkers indicate whether a drug will be effective in a specific patient and how the patient's body will process it. The skilled person is aware of numerous novel biomarkers used in the various and highly specified medical fields.

In another preferred embodiment of the present invention the biomarker is selected from the group selected from nucleic acids, proteins and metabolites. A biomarker may thus refer to a molecule, preferably a protein, measured in blood whose concentration reflects the severity or presence of some disease state. Thus, a biomarker can be used as an indicator of a particular disease state or also a physiological state of an organism.

In another preferred embodiment of the present invention the biomarker molecule is a coagulation factor. It will be appreciated that the one or more biomarker to be tested in the assay according to invention depends on the intended diagnostic purpose. For example, the biomarker may be one which is completely unrelated to the diagnosis of coagulation activity, for instance, a cardiac biomarker such as Troponin-I.

In some further specific embodiments of the present invention the biomarker may be related to coagulation activity. In such a case, it may be useful that the biomarker molecule detected by the optomagnetic measurement according to the present invention is a coagulation factor. The combinatorial measurement of coagulation activity by means of clotting time and the presence/and or amount of one or more coagulation factor can provide a global and comprehensive picture for further diagnosis and prognosis in clinical practice.

Without being limited thereto, suitable coagulation factors that can be used as a biomarker are, for instance, I (fibrinogen) , II (prothrombin), Tissue factor, calcium, V (proaccelerin, labile factor), VI, VII (stable factor, proconvertin), VIII (Antihemophilic factor A), IX (Antihemophilic factor B or Christmas factor), X (Stuart-Prower factor), XI (Plasma thronboplastin antecedent), XII (Hageman factor), XIII (fibrin-stabilizing factor), von Willebrand factor, prekallikrein (Fletcher factor), high-molecular-weight kininogen (HMWK) (Fitzgerald factor), fironection, antithrombin III, heparin cofactor II, protein C, protein S, protein Z, protein Z-related proteae inhibitor (ZPI), plasminogen, alpha 2-antiplasmin, tissue plasminogen activator (tPA), urokinase, plasminogen activator inhibitor-1 (PAI1), plasminogen activator inhibitor-2 (PAI2), and cancer procoagulant, or a combination thereof. The coagulation factor may be detected in their active and non-active from, i.e. degraded or degenerated components. Particular preferred examples of coagulation factors are factors selected from the group consisting of I, II, Xia, XIIa, D-dimer, von Willebrand factor, VIIa, beta-thromboglobulin and Protein C, and Protein S, or a combination thereof.

In further preferred embodiments, the experimental setup as described in Example 3 can be applied for the detection of coagulation-related biomarker. In order to obtain a global and comprehensive picture of coagulation activity as a whole, the antibodies used for detection, i.e. which are either conjugated to the magnetic beads and attached to the sensor surface, may be directed against any coagulation specific factor (active or inactive form) as a further measure of coagulation activity in addition to clotting time.

One particular preferred example for a relevant diagnostic measurement is shown in Example 4, wherein the measurement of clotting time and simultaneous determination of the presence of a cardiac marker and the presence of the coagulation biomarker D-Dimer is carried out. Such a combination of assay steps as envisaged by the present invention shall illustrate the advantages of simultaneous detection as described herein. For instance, D-dimer concentration is typically determined for the diagnosis of thrombosis. In particular, D-dimer testing is of particular clinical use, especially when there is a suspicion of deep venous thrombosis (DVT), pulmonary embolism (PE) or disseminated intravascular coagulation (DIC). The combinatorial test method as described in this example may be helpful to obtain a whole picture for diagnosis of thrombosis in conjunction with other relevant diseases within a short period of time.

In a further preferred embodiment of the present invention the second binding molecule is an antibody or a fragment thereof. Preferred examples of a second binding molecule are antibodies or antibody fragments or variants as defined herein above, which specifically bind a target molecule, preferably a biomarker molecule captured by a first binding molecule. In the case of antibody second binding molecules, the antibodies may bind to different epitopes of a target molecule than the first binding molecule, or may detect an epitope generated by a specific binding of the target molecule to a first binding molecule.

In yet a further preferred embodiment of the present invention the presence and/or amount of the at least one target molecule is detected using a sandwich immunoassay.

The following examples and figures are provided for illustrative purposes. It is thus understood that the example and figures are not to be construed as limiting. The skilled person in the art will clearly be able to envisage further modifications of the principles laid out herein.

EXAMPLES

*Example 1* - *Measurement of coagulation activity by determining clotting time of a plasma sample using an optomagnetic assay.*

The coagulation cascade in plasma typically results in formation of fibrin networks which hamper the movement of magnetic nanoparticles in the liquid to be tested. An exemplary measurement of clotting time according to the invention is carried out on the Magnotech®-biosensor system using a detection based on frustrated total internal reflection (FTIR). The experimental setup is depicted in Fig. 1. Magnetic actuation of the magnetic particles occurs via the alternate action of two magnets, i.e. an upper magnet (washing magnet) and a lower magnet (binding magnet). The clotting time in this example is measured utilizing a standard Magnotech® biosensor cartridge for FTIR detection and 500 nm superparamagnetic beads. Likewise the beads may be already provided within a biosensor cartridge or added together with the blood sample mixture.

In this Example, a mixture of 5 $\mu$l citrate plasma, 5 $\mu$l 0.125 M $CaCl_2$ and 10 $\mu$l 500nm superparamagnetic beads (2 mg/ml) is injected into a sample cartridge. In a control experiment, the same mixture without the addition of 0.025 M $CaCl_2$ is injected into the cartridge. The addition of calcium serves to activate the coagulation cascade in a blood sample which contains citrate as anticoagulant for prevention of clotting. In the control experiment (Fig. 2a) where no calcium is added, the sample thus corresponds to a sample where the coagulation cascade is inhibited or where the fibrin formation is not critical or sufficient to inhibit the movement of the magnetic particles.

The magnetic actuation consists of a phase where the beads are pulled towards and away from the surface in a pulse-like fashion. Due to the creation of an evanescence field by FTIR the beads only become visible when trapped or immobilized close or at the sensor surface thereby creating a high contrast in the FTIR image. In contrast, magnetic beads which are not compromised in their movement can move freely and frequently due to the magnetic actuation and do thus not result in a signal change in the FTIR image. As can be seem from Figure 2A the beads move freely through the citrate plasma in absence of calcium indicating that the formation of fibrin at each of the time points 0,3 and 7 minutes (after injection) is not critical for movement of the beads.

In the control experiment, 0.125 M $CaCl_2$ was added to activate the coagulation cascade in citrated plasma. As can be seen is Figure 2B the right panel shows a gray spot with high contrast (decreased light signal). This change in light signal can be measured after about 4 minutes (see Figure 3) and corresponds to those beads magnetic beads which become trapped close to the sensor surface by the creation of a fibrin network.

The images are analyzed by measuring the gray signal. Figure 3 is a diagram showing the FTIR measurement of gray signal over time. While the curve with squares (without calcium) remains equal over time, the curve with triangles(with calcium) shows a strong change in light signal at about 4 minutes after injection. The time period between injection (time point 0) and change of light signal is herein referred to as the clotting time.

*Example 2 - Measurement of coagulation activity by determining clotting time of a whole blood sample using an optomagnetic assay*

The same measurement as exemplified in Example 1 can be carried out using a whole blood sample instead of plasma. Although whole blood sample comprises blood cells, preliminary tests have shown that the free movement of the magnetic particles are not compromised by the presence of hematocrit. Using the experimental setup as shown in Fig. 1, 5 $\mu$l of a whole blood sample is injected into the sample container already comprising the 500 nm superparamagnetic beads and the change in light signal is measured over time. The time period between injection (time point 0) and change in light signal corresponds to the clotting time and serves as measure of coagulation activity.

*Example 3 - Simultaneous measurement of coagulation activity by determining clotting time of a citrated blood sample and of the presence/amount of a coagulation unrelated biomarker Troponin-I using an optomagnetic assay*

Example 1 and 2 demonstrate that coagulation activity can be successfully measured using an optomagnetic device as described in Figure 1. In order to assess whether a simultaneous measurement using the same cartridge comprising superparamagnetic beads functionalized with a binding molecule directed against a coagulation unrelated biomarker within the blood sample is feasible, 5 $\mu$l citrated plasma is injected with or without 5 $\mu$l 0.125 M CaCl$_2$ in PBS into a sample container containing 500 nm superparamagnetic beads which surface is covered with an antibody directed against the biomarker Troponin-I. Immediately after injection magnetic actuation is started and the biomarker is detected as a round spot on the cartridge surface within about 2 to 3 minutes after injection. The measurement of clotting is measured immediately thereafter at 3 to 7 min.

*Example 4 - Simultaneous measurement of coagulation activity by determining clotting time of a citrated blood sample and of the presence/amount of a coagulation related biomarker such as D-dimer using an optomagnetic assay*

The same experimental setup as described in Example 3 can be also applied for the detection of a coagulation-related biomarker. In order to be able to diagnose and detect coagulation activity as a whole the antibodies conjugated to the magnetic beads may be directed against any coagulation specific factor (active or inactive form) as a further measure of coagulation activity in addition to clotting time. One example for a relevant diagnostic measurement is, e.g. the measurement of clotting time and simultaneous determination of the presence of a cardiac marker and the presence of the coagulation biomarker D-Dimer, which is a fibrin degradation product. D-dimer concentration is typically determined for diagnosis of thrombosis. The combinatorial assay as described in this example thus serves to obtain a global picture of coagulation activity in combination with a coagulation-related and a coagulation-unrelated marker.

**Claims**

1.  Method for simultaneous detection of the coagulation activity of a blood sample and of the presence and/or amount of at least one target molecule within said blood sample, comprising the steps of:

    i) introducing a blood sample into a sample container comprising magnetic particles and a sensor surface, wherein said magnetic particles are functionalized with a first binding molecule, wherein said first binding molecule is attached to said magnetic particles, wherein the first binding molecule is capable of specifically binding to the at least one target molecule within said blood sample, and wherein a second binding molecule is attached to a sensor surface at the bottom of said sample container, and wherein said second binding molecule of the sensor surface is capable of specifically binding to said at least one target molecule within said blood sample;
    ii) measuring the presence and/or amount of said target molecule by detecting the number of magnetic particles bound to said sensor surface; wherein the number of bound magnetic particles is directly or inversely related to the amount of said at least one target molecule present in the sample;
    iii) at the same time or at different times measuring the coagulation activity of said blood sample, wherein said magnetic particles are magnetically actuated; and
    wherein the loss of mobility of said magnetic particles is detected by measuring the light reflected from said immobilized magnetic particles near or at said sensor surface; and
    wherein the change of detected light signal is indicative of an increase of viscosity and/or a clotting of the blood sample; and
    wherein the magnetic particles in steps i) to iii) are magnetically actuated using a magnetic field generator.

2. The method of claim 1, wherein the detection of the coagulation activity of a blood sample and of the presence and/or amount of at least one target molecule within said blood sample is carried out using frustrated total internal reflection (FTIR).

3. The method claim 1 or 2, wherein the steps i) to iii) are carried out in the same sample container.

4. The method of claim 3, wherein the steps i) to iii) are carried using the same magnetic particles, the magnetic particles preferably be superparamagnetic beads.

5. The method of any of claims 1 to 4, wherein said first binding molecule is an antibody or a fragment thereof.

6. The method of any of claims 1 to 5, wherein the blood sample is taken from an individual, and wherein the blood sample is an untreated whole blood sample or a blood-derived sample.

7. The method of any of claims 1 to 6, wherein the magnetic particles in step iii) are actuated in such a way that the beads are pulled towards and away from the surface in a pulse like fashion.

8. The method of any of claims 1 to 7, wherein the light reflected from the magnetic particles in step iii) is measured over time, wherein introduction of the blood sample is designated as the starting point and said drop of detected light signal is designated as the endpoint, and wherein the period between said starting point and end point correlates to the clotting time.

9. The method of any of claims 1 to 8, wherein the coagulation activity is induced by the addition of a further substance into the sample container.

10. The method of any of claims 1 to 9, wherein said at least one target molecule is a biomarker for diagnosis and/or course of diseases.

11. The method of claim 10, wherein said biomarker is selected from the group selected from nucleic acids, proteins and metabolites.

12. The method of any of claims 10 or 11, wherein the biomarker molecule is a coagulation factor.

13. The method of any of claims 1 to 12, wherein said second binding molecule is an antibody or a fragment thereof.

14. The method of any of claims 1 to 13, wherein said presence and/or amount of the at least one target molecule is detected using a sandwich immunoassay.

FIGURE 1

FIGURE 2

FIGURE 2A

FIGURE 2B

FIGURE 3

Europäisches
Patentamt

European
Patent Office

Office européen
des brevets

# EUROPEAN SEARCH REPORT

Application Number

EP 11 18 7673

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DITTMER WENDY U ET AL: "Rapid, high sensitivity, point-of-care test for cardiac troponin based on optomagnetic biosensor", CLINICA CHIMICA ACTA, vol. 411, no. 11-12, June 2010 (2010-06), pages 868-873, XP002666148, ISSN: 0009-8981 * abstract; figures 1, 2, 3 * | 1-14 | INV. G01N33/543 G01N33/86 G01N33/49 |
| A | US 6 165 795 A (MIZE PATRICK D [US] ET AL) 26 December 2000 (2000-12-26) * abstract; claim 13 * | 1-14 | |
| A | WO 2005/106467 A1 (MEDTRONIC INC [US]; WRIGHT DAVID W [US]; KRIEWALL TIMOTHY J [US]; CHEE) 10 November 2005 (2005-11-10) * abstract * | 1-14 | |
| A | OBERHARDT B J ET AL: "DRY REAGENT TECHNOLOGY FOR RAPID, CONVENIENT MEASUREMENTS OF BLOOD COAGULATION AND FIBRINOLYSIS", CLINICAL CHEMISTRY, AMERICAN ASSOCIATION FOR CLINICAL CHEMISTRY, WASHINGTON, DC, vol. 37, no. 4, 1 April 1991 (1991-04-01), pages 520-526, XP000371778, ISSN: 0009-9147 * abstract * * page 524, left-hand column, paragraph 2 - right-hand column, paragraph 1 * | 1-14 | TECHNICAL FIELDS SEARCHED (IPC) G01N |

The present search report has been drawn up for all claims

| Place of search | Date of completion of the search | Examiner |
|---|---|---|
| Munich | 20 December 2011 | Luis Alves, Dulce |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons

& : member of the same patent family, corresponding
     document

EPO FORM 1503 03.82 (P04C01)

**ANNEX TO THE EUROPEAN SEARCH REPORT
ON EUROPEAN PATENT APPLICATION NO.**

EP 11 18 7673

This annex lists the patent family members relating to the patent documents cited in the above-mentioned European search report.
The members are as contained in the European Patent Office EDP file on
The European Patent Office is in no way liable for these particulars which are merely given for the purpose of information.

20-12-2011

| Patent document cited in search report | | Publication date | Patent family member(s) | | Publication date |
|---|---|---|---|---|---|
| US 6165795 | A | 26-12-2000 | AU | 763074 B2 | 10-07-2003 |
| | | | AU | 4954699 A | 10-01-2000 |
| | | | BR | 9911467 A | 20-03-2001 |
| | | | CA | 2335825 A1 | 29-12-1999 |
| | | | EP | 1088223 A1 | 04-04-2001 |
| | | | JP | 2002519635 A | 02-07-2002 |
| | | | JP | 2008224687 A | 25-09-2008 |
| | | | TW | 536623 B | 11-06-2003 |
| | | | US | 6165795 A | 26-12-2000 |
| | | | WO | 9967630 A1 | 29-12-1999 |
| WO 2005106467 | A1 | 10-11-2005 | EP | 1740944 A1 | 10-01-2007 |
| | | | US | 2005233466 A1 | 20-10-2005 |
| | | | WO | 2005106467 A1 | 10-11-2005 |

EPO FORM P0459

For more details about this annex : see Official Journal of the European Patent Office, No. 12/82

**REFERENCES CITED IN THE DESCRIPTION**

**Patent documents cited in the description**

- US 4876069 A
- WO 9967630 A
- WO 2008155716 A
- EP 1801594 A
- WO 2007010469 A1